# EUROPEAN PATENT APPLICATION

(11) **EP 1 500 389 A1**
(43) Date of publication of application: **26.01.2005**
(21) Application number: 04017202.5
(22) Date of filing: 21.07.2004
(51) Int. Cl.: A61K 9/00, A61P 31/04

(54) **Biofilm removing agent**

(30) Priority: 24.07.2003 JP 2003279372
(71) Applicant: Matsumoto Dental University, Nagano 399-0781 (JP)
(72) Inventor: Wang, Pao-Li, Hirooka, Shiojiri Nagano 399-0781 (JP)
(74) Representative: VOSSIUS & PARTNER

(57) **Abstract**

In a biofilm removing agent containing a macrolide antibiotic for removing a biofilm formed by periodontal pathogens, the macrolide antibiotic is 15-membered ring azithromycin.

## Description

This application claims priority to prior Japanese application JP 2003-279372, the disclosure of which is incorporated herein by reference.

This invention relates to a biofilm removing agent for removing a biofilm formed by periodontal pathogens to promote drug penetration into a periodontal tissue.

A periodontal disease is known as a problem associated with an inflammatory response induced in a parodontium or periodontium by enzymes and toxic substances released from periodontal pathogens. The inflammatory response leads to progress of destruction of a pericemental membrane and absorption of an alveolar bone.

As a pathologic condition of the periodontal disease, inflammation of gingiva (gingivitis) is observed at first. Next, a periodontal pocket is formed in the gingiva. Thereafter, absorption of the alveolar bone progresses and a tooth is loosened. Eventually, the tooth is lost.

In the periodontal disease, plaque is at first formed on a gingival margin. Specifically, the plaque is deposited and accumulated on the gingival margin by gram-positive bacteria. In a lower part of the plaque, oxygen is consumed in a process of accumulation of the gram-positive bacteria, resulting in lack of oxygen. Such oxygen-lacking environment promotes the growth of gram-negative bacteria regarded as the periodontal pathogens.

Therefore, beneath the gingival margin, the gram-negative bacteria predominantly grow and form a community to cause destruction of the periodontal tissue and promote deterioration of the pathologic condition. Thus, a combination of the gram-positive bacteria on the gingival margin and the gram-negative bacteria beneath the gingival margin forms a complex population of bacteria.

Some species of the gram-negative bacteria are believed to secrete a viscous substance. It is supposed that, in a growth process of such bacteria, a biofilm as a layer of the viscous substance is formed.

A common clinical feature of biofilm infections is said to be a refractory disease. In an oral cavity, a community of free-floating bacteria pathogenic for the periodontal tissue is released from the biofilm in the periodontal pocket and adhered to the periodontal tissue to penetrate into and damage the periodontal tissue. When a large amount of biofilms are accumulated and a resistance of the periodontal tissue is weakened, this leads to onset or progress of the periodontal disease.

In a dental clinic, the biofilm beneath the gingival margin is removed by a physical treatment and the periodontal pathogens are removed by a medical agent.

The plaque beneath the gingival margin forms the biofilm so that the medical agent is difficult to reach. In order to enable the medical agent to effectively act upon the biofilm, the medical agent must be dosed at a high concentration and held in the periodontal pocket for a predetermined time period.

In a drug therapy used in treatment of the periodontal disease, a local drug delivery system (LDDS) is effective in which dynamic behavior of the medical agent locally dosed is controlled.

In LDDS in the treatment of the periodontal disease, the medical agent directly dosed into the periodontal pocket is gradually released so as to maintain an effective concentration and to control the periodontal pathogens in the periodontal pocket.

The LDDS is advantageous in that the medical agent of a very high concentration, which can not be achieved by systemic administration, is maintained for a predetermined time period while a systemic side effect is suppressed.

As a medical agent for removing tetracycline-resistant staphylococci as the periodontal pathogens involved in inflammation caused in the periodontal tissue, minocycline hydrochloride as a tetracycline derivative is known. Minocycline exhibits antibacterial activity against free-floating bacteria released from the biofilm (for example, see Japanese Patent Application Publication (JP-A) No. H11-292767).

As a medical agent for destroying the biofilm formed in the periodontal pocket, macrolide antibiotics are known. The macrolide antibiotics are effective also for preventing formation of the biofilm.

The macrolide antibiotics are classified into 14- to 16-membered ring macrolide antibiotics depending upon the number of atoms forming a lactone ring. For example, erythromycin and clarithromycin as 14-membered ring macrolide antibiotics and josamycin as a 16-membered ring macrolide antibiotic are known. Among others, the 14-membered ring macrolide antibiotics are known to be useful for preventing formation of the biofilm and for removing the biofilm (for example, see Japanese Patent Application Publication (JP-A) No. H7-267868).

Minocycline hydrochloride as a tetracycline derivative is expected to exhibit an antibacterial effect only against the free-floating bacteria released from the biofilm in the periodontal pocket. However, there is a problem that the biofilm itself is resistant against minocycline.

The macrolide antibiotics are expected to have a certain inhibition ratio and a certain destruction ratio for the biofilm. As a biofilm removing agent, however, it is desired to develop a medical agent which can be safely administered for a long time and which exhibits a higher destruction ratio and a higher inhibition ratio.

It is therefore an object of this invention to provide a biofilm removing agent which can be safely administered for a long time and which is capable of removing a biofilm with a high destruction ratio.

It is another object of this invention to provide a biofilm removing agent which can be safely administered for a long time and which is capable of inhibiting formation of a biofilm with a high inhibition ratio.

According to this invention, there is provided a biofilm removing agent containing a macrolide antibiotic for removing a biofilm formed by periodontal pathogens, wherein the macrolide antibiotic is 15-membered ring azithromycin.

### Brief Description of the Drawing:

Fig. 1 is a graph showing a biofilm destruction ratio of a biofilm removing agent containing azithromycin according to this invention in comparison with minocycline, erythromycin, and josamycin; and
Fig. 2 is a graph showing a biofilm inhibition ratio of the biofilm removing agent containing azithromycin according to this invention in comparison with minocycline, erythromycin, and josamycin.

### Description of the Preferred Embodiment:

A biofilm removing agent according to this invention contains a macrolide antibiotic which is 15-membered ring azithromycin.

Hereinafter, a biofilm removing agent according to an embodiment of this invention will be described in conjunction with specific examples.

The biofilm removing agent contains a macrolide antibiotic for destroying a biofilm formed by periodontal pathogens. The macrolide antibiotic is 15-membered ring azithromycin.

### Example 1

Periodontal pathogens were put in a glass vessel and left for 24 hours to form a biofilm. Then, a solution of a biofilm removing agent containing 1 mg of azithromycin as a medical agent dissolved in 100ml of distilled water was supplied into the glass vessel. After lapse of 24 hours, a destruction ratio of the biofilm formed by the periodontal pathogens was examined.

Further, the biofilm destruction ratio of the biofilm removing agent containing azithromycin according to this invention was evaluated in comparison with the biofilm destruction ratio of each of minocycline, erythromycin, and josamycin. Specifically, each of minocycline, erythromycin, and josamycin was supplied together with distilled water into a glass vessel in which the biofilm was formed. After lapse of 24 hours, the destruction ratio of the biofilm formed by the periodontal pathogens was examined.

The content of each of minocycline, erythromycin, and josamycin in the distilled water was equal to that of the biofilm removing agent containing azithromycin.

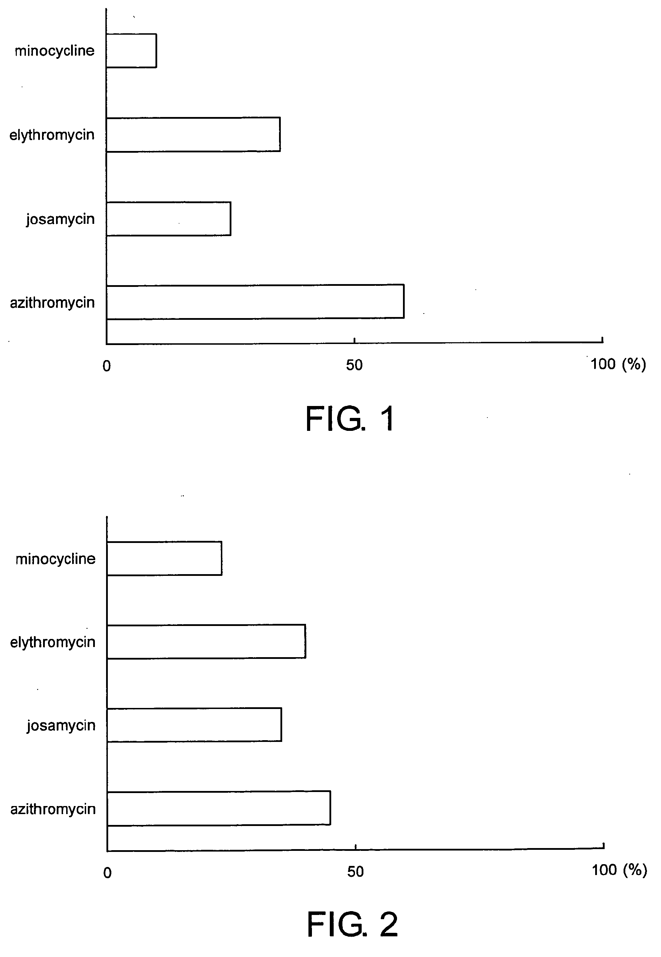

Fig. 1 shows the biofilm destruction ratio (the destruction ratio of gram-negative bacteria) of each of azithromycin, minocycline, erythromycin, and josamycin. In Fig. 1, the biofilm destruction ratio by each of azithromycin, minocycline, erythromycin, and josamycin is a ratio of a destroyed or lost part of the biofilm with respect to a biofilm formed by the periodontal pathogens and supplied with distilled water alone without any antibiotic.

As seen from Fig. 1, the biofilm destruction ratio of the biofilm removing agent containing azithromycin was about 60%. The biofilm destruction ratios of minocycline, erythromycin, and josamycin were about 10%, about 35%, and about 25%, respectively.

Thus, it is verified that the biofilm removing agent containing azithromycin has a biofilm destruction ratio higher than those of minocycline, erythromycin, and josamycin.

### Example 2

Periodontal pathogens were put in a glass vessel together with a biofilm removing agent containing 1 mg of azithromycin as a medical agent dissolved in 100ml of distilled water. After lapse of 24 hours, an inhibition ratio of the biofilm to be formed by the periodontal pathogens was examined.

Further, the biofilm inhibition ratio of the biofilm removing agent containing azithromycin according to this invention was evaluated in comparison with the biofilm inhibition ration of each of minocycline, erythromycin, and josamycin. Specifically, each of minocycline, erythromycin, and josamycin was put into a glass vessel together with periodontal pathogens and distilled water. After lapse of 24 hours, the biofilm inhibition ratio of the biofilm formed by the periodontal pathogens was examined.

The content of each of minocycline, erythromycin, and josamycin in the distilled water was equal to that of the biofilm removing agent containing azithromycin.

Fig. 2 shows the biofilm inhibition ratio as a ratio of inhibiting formation of the biofilm (the adhesion prevention ratio of gram-negative bacteria) by each of azithromycin, minocycline, erythromycin, and josamycin. In Fig. 2, the biofilm inhibition ratio of each of azithromycin, minocycline, erythromycin, and josamycin is a ratio of a part without the biofilm with respect to an entire biofilm formed by the periodontal pathogens in presence of distilled water alone without any antibiotic.

As seen from Fig. 2, the biofilm inhibition ratio of the biofilm removing agent containing azithromycin was about 45%. The biofilm inhibition ratios of minocycline, erythromycin, and josamycin were about 23%, about 40%, and about 35%, respectively.

Thus, it is verified that the biofilm removing agent containing azithromycin has a biofilm inhibition ratio higher than those of minocycline, erythromycin, and josamycin.

As verified in conjunction with Figs. 1 and 2, the biofilm destruction ratio and the biofilm inhibition ratio achieved by azithromycin are remarkably higher than those achieved by minocycline, erythromycin, and josamycin. Thus, it is found out that azithromycin exhibits an excellent effect of destructing the biofilm and inhibiting formation of the biofilm as compared with other medical agents.

The biofilm removing agent containing azithromycin is excellent in tissue migration, in particular, migration to an infected tissue. Further, the biofilm removing agent containing azithromycin has a serum concentration half-life or a tissue concentration half-life as long as 60 to 80 hours. Therefore, administration of three doses per day for three days achieves sufficient clinical efficacy.

Azithromycin is excellent in antibacterial action against gram-negative bacteria and is equivalent in induced resistance to 14-memberd ring macrolide antibiotics. Against gastric acid, azithromycin is considerably stable as compared with 14-membered ring macrolide antibiotics. For a digestive side effect, azithromycin is equivalent in effect to 14-membered ring macrolide antibiotics. For activation of hepatic metabolic enzymes, azithromycin has an extremely small level as compared with 14-membered ring macrolide antibiotics.

In order to administer the biofilm containing azithromycin, an ointment or a film containing azithromycin may be directly administered into a periodontal pocket so as to gradually release azithromycin. Alternatively, a liquid medicine, such as a mouth wash, containing azithromycin may be used.

The dose of azithromycin as the macrolide antibiotic depends on the mode of administration or the degree of disease. Generally, the dose preferably falls within a range of 0.5-10 mg/day, more preferably 1-5 mg/day. The dose within the above-mentioned range is lower than that intended for typical sterilization and will not cause any safety problem such as a side effect.

In order to use the biofilm removing agent according to this invention as a liquid mouth wash, azithromycin (5 wt%) is dissolved in ethanol (65 wt%) and purified water (30 wt%) is added thereto.

In order to use the biofilm removing agent according to this invention as a liquid ointment, dense glycerin (83 wt%) and hydroxyethyl cellulose (3wt%) are mixed and kneaded to obtain a nonaqueous gel. To the nonaqueous gel, azithromycin (2 wt%), aminoalkyl methacrylate copolymer RS (2 wt%), triacetin (10 wt%) are added. Thus, the ointment having a desired sustained-release effect is obtained. The ointment is filled in a syringe adapted for administration into the periodontal pocket and injected into the periodontal pocket as an affected site.

In order to use the biofilm removing agent according to this invention as a liquid film, a drug layer and a support layer are prepared.

To produce the drug layer, carboxyvinyl polymer (20 wt%), hydroxypropylmethyl cellulose (30 wt%), and polyvinyl acetic acid (50 wt%) are swelled in water to obtain a stock gel. An appropriate amount of the stock gel is mixed with azithromycin (2 wt%) dissolved in ethanol. To an appropriate amount of the stock gel, azithromycin (2 wt%) dissolved in ethanol is added. A resultant gel is extended, dried, and then cut. A drying temperature is 75 °C and a drying time is 10 minutes.

Separately, the support layer is produced. A methanol solution of polyvinyl acetic acid is prepared, extended on a release paper, and dried. Thereafter, the support layer formed on the release paper is cut. A drying temperature is 75 °C and a drying time is 8 minutes.

The drug layer and the support layer are thermocompression-bonded by a pressing iron and cut into an appropriate size to obtain a desired film. A thermocompression-bonding temperature is 110 °C and a thermocompression-bonding time is 30 seconds.

The biofilm removing agent according to this invention may be used in various manners. The liquid mouth wash is injected into the oral cavity. The ointment is filled in the syringe adapted to administration into the periodontal pocket and is injected into the periodontal pocket as the affected site. The film may be adhered to gingiva or inserted into the periodontal pocket of a patient.

Azithromycin used in this invention is high in inhibition ratio of inhibiting formation of the biofilm by the periodontal pathogens and in destruction ratio of destroying the biofilm. Therefore, various medicinal agents can be directly penetrated into and acted upon the affected part.

According to this invention, formation of the biofilm is inhibited and the biofilm is destroyed by azithromycin and the medicinal agents can effectively be penetrated into and acted upon the affected part.

While this invention has thus far been described in conjunction with the preferred embodiment and the specific examples thereof, it will be readily possible for those skilled in the art to put this invention into practice in various other manners without departing from the scope of this invention.

## Claims

1. A biofilm removing agent containing a macrolide antibiotic for removing a biofilm formed by periodontal pathogens, wherein the macrolide antibiotic is 15-membered ring azithromycin.

2. A biofilm removing agent according to claim 1, wherein said biofilm removing agent contains ethanol and purified water.

3. A biofilm removing agent according to claim 1 or 2, wherein said biofilm removing agent contains hydroxyethyl cellulose, aminoalkyl methacrylate copolymer RS, triacetin, and glycerin.

4. A biofilm removing agent according to claim 1, 2 or 3, wherein said biofilm removing agent contains carboxyl vinyl polymer, polyvinyl acetic acid, hydroxypropylmethyl cellulose, and sodium citrate.
